# EUROPEAN PATENT APPLICATION

(11) **EP 0 594 877 A1**
(43) Date of publication of application: **04.05.1994**
(21) Application number: 92118254.9
(22) Date of filing: 26.10.1992
(51) Int. Cl.: C07D 487/04, C07D 519/00, A61K 31/505

(54) **Imidazo(1,2-c)quinazoline derivates as antihyper tensives and anti dysurics**

(71) Applicant: NATIONAL SCIENCE COUNCIL, Taipei (TW)
(72) Inventor: Chern, Ji-Wang, Taipei (TW); Lu, Guan-Yu, Taipei (TW); Shiau, Chia-Yang, 5F, No.16-3, 25 Alley, Taipei (TW); Yen, Mao-Hsiung, Taipei (TW)
(74) Representative: Grünecker, Kinkeldey, Stockmair & Schwanhäusser Anwaltssozietät

(57) **Abstract**

Compounds of formula 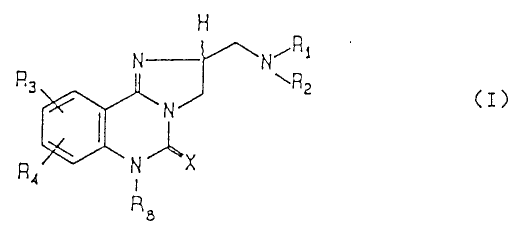
wherein:
X is O or S;
R₁ is hydrogen, alkyl, phenyl or phenyl(alkyl);
R₂ is hydrogen, alkyl, phenyl or phenyl(alkyl); or R₁ and
R₂ together represent alkylene or 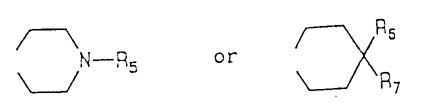
wherein R₅ represents (a) 2-(2-hydroxyethoxy)ethyl; (b) pyrimidinyl; (c) a 2-, 3- or 4-pyridinyl optionally substituted by alkyl; and (d) benzoyl, benzyl,
phenyl and diphenylmethyl optionally substituted
by halogen, methoxy, trifluoromethyl, alkyl, nitro, acetyl, and cyano; and
(i) R₆ and R₇ together represent alkylene or (ii) 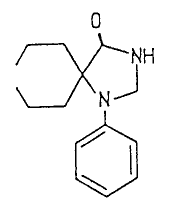
or (iii) when R₆ represents hydrogen atom or hydroxy, R₇ represents (a) pyrimidinyl; (b) a 2-, 3- or 4-pyridinyl optionally substituted by alkyl; and (c) benzoyl, benzyl, phenyl and diphenylmethyl optionally substituted halogen, methoxy, trifluoromethyl, alkyl, nitro, acetyl, and cyano;
R₃ is halogen, hydrogen, alkyl, trifluoromethyl, or alkoxy;
R₄ is halogen, hydrogen, alkyl, trifluoromethyl, or alkoxy; and
R₈ is hydrogen, alkyl or phenyl(alkyl).

These compounds are found useful as an active ingredient for the treatment of hypertension and dysuria.

## Description

### Technical Field of the Invention

The present invention relates to new and useful imidazo[1,2-c]quinazolinone derivatives, especially relates to 2-substituted methyl-2,3-dihydroimidazo[1,2-c]quinazolin-5-ones (-thiones).

### Background of the Invention

Quinazoline ring system constituting a class of antihypertensive agents is well known in the art. Prazosin disclosed in U.S. Patent 3,511,836, a 2-substituted quinazoline derivative, has been proven effective in the clinic acting as an α₁-adrenoceptor antagonist.

U.S. Patent 4,335,127 discloses 3-substituted quinazolines such as ketanserin, thioketanserin which have been found being potent serotonin-antagonists.

In an article entitled "Further Evaluation of the Selectivity of a Novel Antihypertensive agent, SGB-1534, for "Peripheral α₁-adrenoceptors in the Spinally Anesthetized Dog" Eur. J. Pharmacol. 1986, 131, 257-264, Junichi Imagawa et al. disclose SGB-1534, a 3-substituted quinazoline derivative, which also have been proven to have antihypertensive activities by an α₁-adrenoceptor antagonist.

Recently, it was reported in Drug of the Future 1989, 14, 400 and Brit. J. Pharmacol. 1988, 93, 702-14 that α₁-adrenoceptor antagonists can be used for the treatment of dysuria which is due to the prostatauxe.

As a result of an extensive investigations to develop a more potent and clinically effective antihypertensive and anti-dysuria agent, a novel series of imidazo[1,2-c]quinazolinone derivatives, 2-substituted methyl-2,3-dihydroimidazo[1,2-c]quinazolin-5-ones (-thiones), have been newly synthesized in the present invention.

Accordingly, one object of the present invention is to provide new and useful 2-substituted methyl-2,3-dihydroimidazo[1,2-c]quinazolin-5-ones (-thiones) and pharmaceutically acceptable salts thereof.

Another object of the present invention is to provide a process for preparing the 2-substituted methyl-2,3-dihydroimidazo[1,2-c]quinazolin-5-ones (-thiones) and pharmaceutically acceptable salts thereof.

Another object of the present invention is to provide a pharmaceutical composition containing, as an active ingredient, the 2-substituted methyl-2,3-dihydroimidazo[1,2-c]quinazolin-5-ones (-thiones) or a pharmaceutically acceptable salt thereof.

Another object of the present invention is to provide a method for the treatment of high blood pressure comprising adminstrating to a mammal in need of said treatment an effective amount of the 2-substituted methyl-2,3-dihydroimidazo[1,2-c]quinazolin-5-ones (-thiones) or a pharmaceutically acceptable salt thereof.

A futher object of this invention is to provide a method for the treatment of dysuria comprising adminstrating to a mammal in need of said treatment an effective amount of the 2-substituted methyl-2,3-dihydroimidazo-[1,2-c]quinazolin-5-ones (-thiones) or a pharmaceutically acceptable salt thereof.

### Summary of the Invention

The present invention provide a novel series of 2-substituted methyl-2,3-dihydroimidazo[1,2-c]quinazolin-5-ones (-thiones) compounds having the formula: 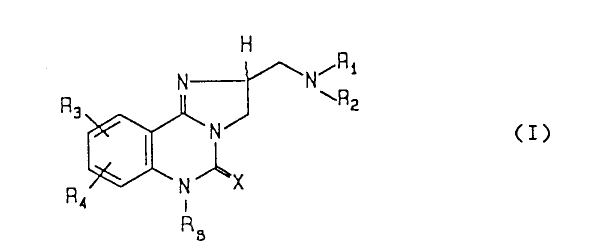
wherein:
X is O or S;
R₁ is hydrogen, lower alkyl, phenyl or phenyl(lower alkyl);
R₂ is hydrogen, lower alkyl, phenyl or phenyl(lower alkyl); or R₁ and R₂ together represent alkylene of 4 to 10 carbon stoms or 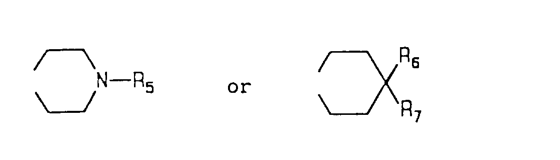
wherein R₅ represents (a) 2-(2-hydroxyethoxy)ethyl; (b) pyrimidinyl; (c) a 2-, 3- or 4-pyridinyl optionally substituted by one or two substituents each independendly selected from the group consisting of C₁-C₄ alkyl; and (d) benzoyl, benzyl, phenyl and diphenylmethyl optionally substituted by one or two substituents selected from the group consisting of halogen atom, methoxy, trifluoromethyl, C₁-C₄ alkyl, nitro, acetyl, and cyano; and
(i) R₆ and R₇ together represent alkylene of 4 to 6 carbon atoms or
(ii) 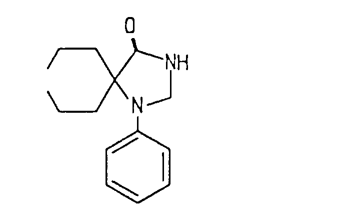
or (iii) when R₆ represents hydrogen atom or hydroxy, R₇ represents (a) pyrimidinyl; (b) a 2-, 3- or 4-pyridinyl optionally substituted by one or two substituents each independendly selected from the group consisting of C₁-C₄ alkyl; and (c) benzoyl, benzyl, phenyl and diphenylmethyl optionally substituted by one or two substituents selected from the group consisting of halogen atom, methoxy, trifluoromethyl, C₁-C₄ alkyl, nitro, acetyl, and cyano;
   R₃ is halogen, hydrogen, alkyl, trifluoromethyl, or lower alkoxy;
   R₄ is halogen, hydrogen, alkyl, trifluoromethyl, or lower alkoxy; and
   R₈ is hydrogen, C₁-C₄ alkyl or phenyl(C₁-C₄)alkyl; or
   pharmaceutically acceptable salts thereof.

The compounds represented by formula (I) include racemic mixture of optically active compounds and optically pure R and S stereoisomers.

The compounds of formula (I) and the salts thereof are excellent in antihypertensive activity and are, therefore, useful as pharmaceuticals.

### Brief Description of the Drawings

Fig. 1 is a does-response plot of 2-(4-phenyl-1-piperazinyl)methyl-2,3-dihydroimidazo[1,2-c]-quinazoline-5(6H)-one (Example 4) on blood pressure in 5 cats;
Fig. 2 is a time-course of various doeses of Example 4 compound on blood pressure in cats; and
Fig. 3 is a plot showing antagonistic effect of Example 4 compound (0.25mg/kg) on blood pressures induced by phenylephrine (0.015mg/kg) or serotonin (0.020mg/kg).

### Detailed Description of the Preferred Embodiments

As used in the foregoing definitions term "lower alkyl" is meant to include straight and branched hydrocarbon radicals having from 1 to 6 carbon atoms; the term "alkylene" comprises straight and branched alkylene; and the term "halogen" refers to fluorine, chlorine and bromine.

The pharmacologically allowable salts include inorganic acid salts such as hydrochlorides, sulfates, hydrobromides, perchlorates and nitrates; and organic acid salts such as oxalates, maleates, fumarates, succonates and methanesulfonates.

Preferred compounds within the scope of formula (I) are 2-(4-phenyl-1-piperazinyl)methyl-2,3-dihydroimidazo[1,2-c]quinazolin-5(6H)-one; 2-(4-phenyl-1-piperazinyl)methyl-2,3-dihydroimidazo[1,2-c]quinazolin-5(6H)-thione; 2-(4-phenyl-1-piperazinyl)methyl-8,9-dimethoxy-2,3-dihydro-imidazo[1,2-c]quinazolin-5(6H)-one; 2-[4-(2-chlorophenyl)-1-piperazinyl]methyl-2,3-dihydroimidazo[1,2 c]quinazolin-5(6H)-one; 2-(4-phenyl-1-piperazinyl)methyl-6-methyl-2,3-dihydroimidazo[1,2-c]quinazolin-5-one; and 2-[4-(2-chlorophenyl)-1-piperazinyl]methyl-6-methyl-2,3-dihydroimidazo[1,2-c]quinazolin-5-one.

A process suitable for preparing the compounds of formula (I) comprises
(a) reacting a compound having the formula 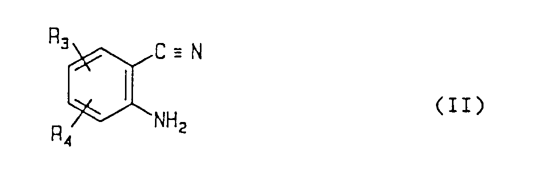 wherein R₃ and R₄ have the same meanings as defined above, with a compound of the formula 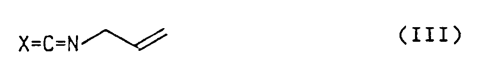 wherein X has the same meaning as defined above;
(b) reacting the resulting compound having the formula 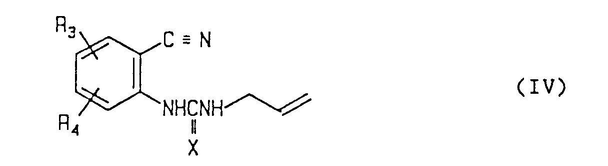 wherein R₃ and R₄ have the same meanings as defined above, with a base;
(c) reacting the resulting compound having the formula 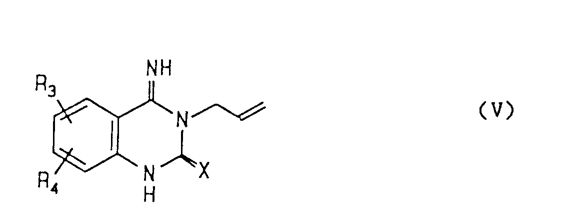 wherein R₃ and R₄ have the same meanings as defined above, with N-halosuccimide or halogen;
(d) reacting the resulting compound having the formula 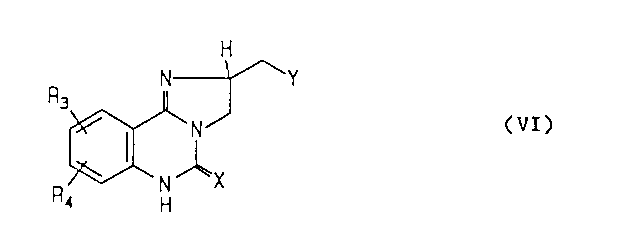 wherein R₃ and R₄ have the same meanings as defined above; Y represents halogen,
   with a compound of formula 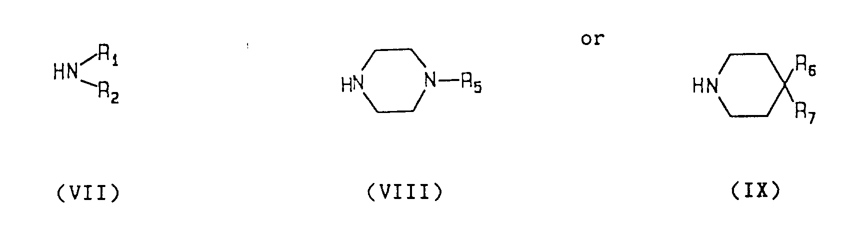 wherein R₁, R₂, R₅, R₆ and R₇ have the same meanings as defined above, as a suitable side chain, to obtain 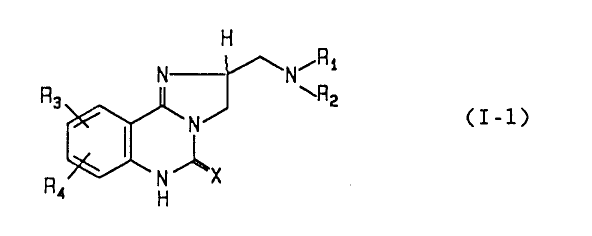 wherein R₁, R₂, R₃, and R₄ have the same meanings as defined above, and
(e) reacting the resulting compound (I-1) with an alkylating agent of formula R₈-Y to obtain 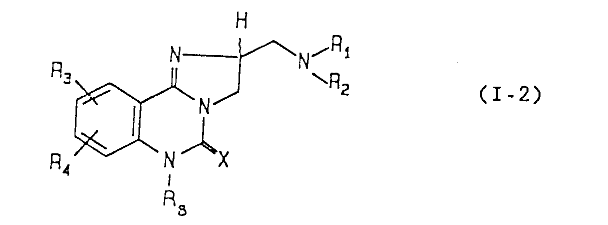 wherein R₈ is C₁-C₄ alkyl or phenyl(C₁-C₄)alkyl; Y is halogen; and R₁, R₂, R₃, and R₄ have the same meanings as defined above.

The reaction between compound (VI) and the appropriate side chain can be carried out in the presence of a suitable base in a suitable solvent. Examples of the suitable base which can be used in the reaction include inorganic bases such as sodium carbonate, potassium carbonate, sodium hydrocarbonate, potassium hydrocarbonate, silver carbonate, cesium carbonate and the like; and the organic bases such as triethyl amine, pyridine, N,N-dimethylaniline, N-methylmorpholine, DBU, DBN and the like. An excess amount of the side chain used in the reaction can also function as a base. The reaction can be carried out in any inert solvent which does not adversely affect the reaction. Examples of these inert solvent include ethers such as diethyl ether, tetrahydrofuran, dioxane, diglyme, etc.; ketones such as MIBK; and acetonitrile. There is no particular limit on the reaction temperature, preferably the reaction temperature is ranging from 0 to 200°C. Generally, the reaction is completed in about 1 to 72 hours.

In the step a), the condensation reaction between the compound of formula (II) and the compound of formula (III) can be carried out neatly or in a suitable solvent. Any solvent which does not interfer with the reactant can be use in the reaction. Examples of suitable solvent include chloroform, dichloromethane, ether, etc. The reaction generally is performed at room temperature and completed in two days.

The ring closure of compound (IV) in the step b) is carried out in the presence of a base such as ammonia water, triethylamine, pyridine, DBN, DBU and the like. The reaction can be carried out in the absence or presence of a solvent. Any inert solvent which does not adversely influence the reaction can be used. Examples of the solvent include water; alcohols such as methanol, ethanol, etc. ; ethers such as diethyl ether, tetrahydrofuran, etc. The reaction generally is completed in about 15 minutes.

The halocyclization of compound (V) in the step c) can be carried out in the presence of N-halosuccimide or halogen in the absence or presence of a suitable solvent. Any solvents which does not influence the reaction can be used such as water, alcohols, ether and dimethylsulfoxide. The reaction usually is carried out at room temperature and completed in about 30 minutes.

The alkylating reaction in step e) is carried out in the presence of a base such as aqueous sodium hydroxide. The reaction is usually completed at room temperature in about three hours.

The compounds of formula (I) and the salts thereof are excellent in antihypertensive activity and are, therefore, useful in the treatment of hypertension.

The potency of the subject compounds as hypertension-antagonists is clearly evidenced by the results obtained in the following tests.

### Antihypertensive Activity

Adult cats of either sex, 2-3 Kg, anesthetized with pentobarbital sodium (30 mg/Kg i.p.) were used. The trachea was intubated to provide artificial respiration with air by means of a Harvard respirator (respiration rate= 16-20/min and colume= 40-50 ml/stroke). Both femoral artery and vein were cannulated with PE 150 to monitor blood pressure and for drug administration respectively. Body temperature was maintained at 37.5°C with a heating pad and monitored with a rectal thermometer. Blood pressure was measured with a Statham P23D pressure transducer via a polyethylene cannula conducted to a right femoral artery. Heart rate was measured through a Grass Model 7B tachograph preamplifier triggered by the pulses of arterial blood pressure. All data were recorded on a Grass Model 7B polygraph. The results are shown in Table 1.

**Table 1**

| Compounds | Dose (mg/Kg) | MBP (mm Hg) | |
|---|---|---|---|
| | | 1 hour | 2 hour |
| Example 4 | 1.0 | -57.5±2.5 | -44.5±0.5 |
| | 0.5 | -49.3±1.5 | -32.5±2.5 |
| | 0.25 | -23.1±2.7 | -8.75±5.7 |
| | 0.125 | -12.5±4.6 | -- |
| Example 7 | 0.25 | -17.5 | -- |
| | 0.5 | -20 | -18.8 |
| | 1.0 | -30 | -15 |
| Example 8 | 4.0 | -43.7±5.2 | -- |
| Example 9 | 4.0 | -30.6±4.7 | -- |
| Example 15 | 4.0 | -30 ±7.3 | -13.8±8.9 |
| Example 17 | 4.0 | -20.3±10.2 | -8.5±2.6 |
| Example 20 | 4.0 | -33.9±9.7 | -22.5±7.6 |
| Example 25 | 4.0 | -38.2±5.7 | -12.6±5.0 |
| Example 26 | 1.0 | -40.7±9.7 | -12.6±3.4 |
| | 0.5 | -25.5±3.4 | -5.7±1.5 |

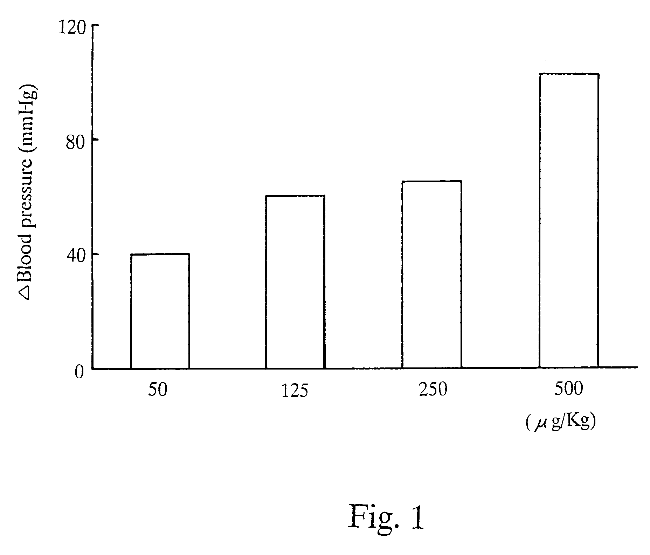

Fig 1. shows the dose-response curves of the maximum changes in SBP, DBP and heart rate after i.v. administration of Example 4 compound in cats. Example 4 compound (10 µg to 1 mg/kg) decreases both SBP and DBP, and changes in heart rate (HR) biphasically (initially increases, then decreases) in a dose-dependent manner, ED₅₀ of lowing blood pressure of Example 4 compound is 250 µg/Kg.

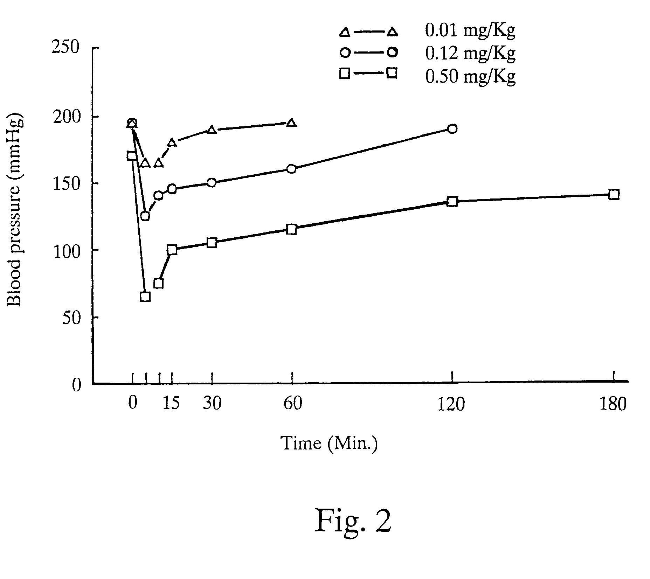

The time course of the hypotensive action of Example 4 compound administration i.v. in anesthetized cat is shown in Fig 2. The solvent (50% DM50) has no significant action on B.P. or H.R. However, Example 4 compound (250 µg/Kg) decreases B.P. immediately and reaches maximally at 5-10 minutes, and the duration of action is about 4 hours.

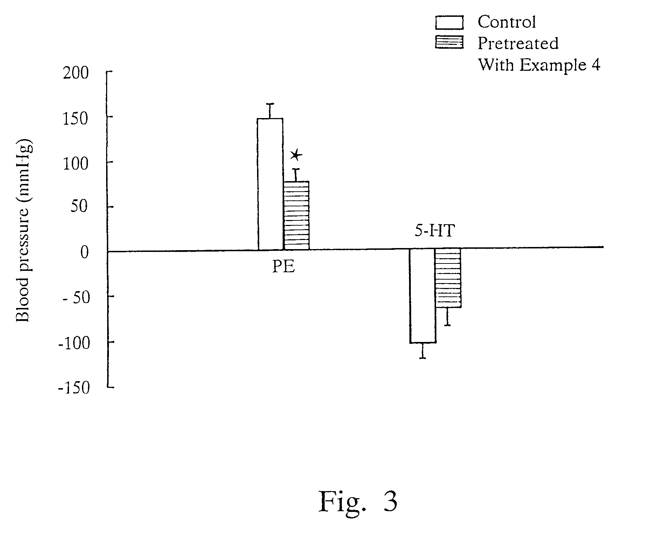

Effects of pretreated with Example 4 compound on blood pressure induced by PE and 5-HT in cats are shown in Fig. 3. The data as shown in Fig. 3 demonstrate that Example 4 compound in doses of ED₅₀ significantly antagonizes the blood pressure response to PE (p<0.02) but no effect on 5-HT (p>0.05). Results suggeste that the imidazo[1,2-c]quinazolinone derivatives of the general formula (I) be a novel α₁-adrenoreceptor antagonist.

### Method of Binding Studies

### 1. Preparation of membranes for binding studies

Rat brain cortex membranes were prepared for [³H] prazosin or [³H] clonidine binding by homogenizing tissues in 0.32 M sucrose buffered with 50 mM Tris buffer (pH 7.4) in a tissue/buffer ratio of 1 : 10. After the removal of nuclei by centrifugation at 1000 x g for 10 min, P₂ membranes were pelleted by centrifuging the supernatant at 22,000 x g for 20 min. After two periods of centrifugation at 22,000 x g and resuspension in fresh buffer, the membrane suspension (about 2 mg/ml protein) was ready for use.

### 2. Binding assays

α₁-Adrenergic receptor binding assays (in triplicate) were carried out with 0.2 nM [³H] prazocin in a final volume of 1.0 ml of Tris buffer at pH 7.4 for 30 min at room temperature, using 10 µM phentolamine to determine non-specific binding. The concentrations of synthetic compounds for competition binding were in the range of 0.1 - 200 nM. α₂-adrenergic receptor binding assays (in triplicate) were carried out with 1 nM [³H] clonidine in the presence of 10 mM MgCl₂ and in a final volume of 1.0 ml of Tris buffer at pH 7.4 for 30 min at room temperature, using 10 µM clonidine to determine non-specific binding. The concentrations of synthetic compounds for competition binding were in the range of 0.1-100 µM. After binding had reached equilibrium, incubations were terminated by collecting the membranes on whatman GF/B filters; the filters were washed twice with 5 ml of 50 mM Tris buffer (pH 7.4) at 4°C. The amount of membrane protein used in each assay was in the range of 300-400 µg, as determined by the method of Lowry et al.. The results are given in Table 2.

### Materials

[³H]prazocin (76.6 Ci/mmol) and [³H]clonidine (47.0 Ci/mmol) were purchased from NEN. All other chemicals used were reagent grade and were purchased from Sigma (St. Louis, Mo).

**Table 2**

| α₁ and α₂-Adrenergic Receptor Binding Affinities for Imidazo[1,2-c]quinazoline Derivatives | | | |
|---|---|---|---|
| Compounds | α₁ Binding (Ki, nM) | α₂ Binding (Ki, nM) | α₂/α₁ ratio |
| Example 4 | 2.01±0.09 | 3860±640 | 1920 |
| Example 5 | 2512±28.1 | -- | -- |
| Example 6 | >100 | -- | -- |
| Example 7 | 1.3±0.5 | 3450±329 | 2654 |
| Example 8 | 850 | >10,000 | >11 |
| Example 9 | 35.8±8.6 | 5011±11 | 140 |
| Example 10 | 95.9±5.2 | -- | -- |
| Example 11 | 14.4 | 391 | 27 |
| Example 12 | 586±9 | -- | -- |
| Example 16 | >100 | -- | -- |
| Example 25 | 30±10 | 1694 | 54 |
| Example 26 | 1.1±0.05 | 3166±396 | 3015 |
| Example 28 | 0.68±0.26 | -- | -- |

Compounds of the invention may be formulated for use in a pharmaceutical composition. Accordingly, in a further aspect of the invention, there is provided a pharmaceutical composition which comprises a compound of formula (I) or pharmaceutically acceptable salt thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such as solid compositions may be used, for example magnesium sterate, starch, lactose, glucose and flavor. The compounds may also be presented with a sterile liquid carrier for injection.

The present invention is now illustrated in greater detail by the following Examples but it should be understood that the present invention is not limited thereto.

### Example 1

### 2-(3-allylureido)benzonitrile

A mixture of anthranilonitrile (10.0 g, 85 mmol) and allyl isocyanate (7.5 mL, 85 mmol) was slightly heated to dissolve. The resulting solution was allowed to stir at room temperature for two days. The solid was collected by filtration and washed with ether (10 mL) to give 14.4 g (100%) of 2-(3-allylureido)benzonitrile. An analytical sample was recrystallized from ethanol. IR (KBr): 3331, 3263, 2226 (CN), 1639 cm⁻¹. ¹H NMR (300 MHz, DMSO-d₆): δ 3.74 (p, 2H, CH₂), 5.09 (q, 1H, =CH), 5.17 (q, 1H, =CH), 5.86 (m, 1H, CH), 7.09 (t, 2H, Ar-H), 7.56 (t, 1H, Ar-H), 7.67 (d, 1H, Ar-H), 8.43 (d, 1H, Ar-H), 7.65 (d, 1H, NH),8.54 (s, 1H, NH). ¹³C NMR (75 MHz, DMSO-d₆): δ 41.47, 101.00, 114.97, 116.99, 120.61, 120.73, 122.22, 132.96, 133.83, 135.69, 142.66, 154.32. Anal. Calcd for C₁₁H₁₁N₃₀ (201.23): C, 65.66; H, 5.51; N, 20.89. Found: C, 65.38; H, 5.51; N, 21.05.

### Example 2

### 3,4-dihydro-4-imino-3-allyl-2(1H)-quinazoline

A mixture of 2-(3-allylureido)benzonitrile (10.0 g, 50 mmol) in ethanol (70 mL) and 28% of ammonia water (50 mL) was heated on a steam bath with occasional stirring for 15 minutes. The mixture was then cooled to room temperature and to the mixture was added water (100 mL). The solid was collected by filtration and was recrystallized from methanol with a few drops of ammonia water to give 8.4 g (84%) of 3,4-dihydro-4-imino-3-allyl-2(1H)-quinazolinone, mp 223-224°C. IR (KBr): 3284, 3203, 3147, 1691, 1582 cm⁻¹. ¹H NMR (300 MHz, DMSO-d₆): δ 4.61 (d, 2H, =CH₂), 5.08 (q, 2H, CH₂), 5.85 (m, 1H, =CH), 7.07 (m, 2H, Ar-H), 7.46 (t, 1H, Ar-H), 8.04 (d, 1H, Ar-H), 8.85 (br s, 1H, NH), 10.74 (s, 1H, NH). ¹³C NMR (75 MHz, DMSO-d₆): δ 42.54, 115.08, 115.81, 122.09, 126.13, 132.77, 133.15, 137.01, 149.76. Anal. Calcd for C₁₁H₁₁N₃O; C, 65.63; H, 5.56; N, 20.87. Found: C, 65.67; H, 5.51; N, 20.86.

### Example 3

### 2-Bromomethyl-2,3-dihydroimidazo[1,2-c]quinazolin-5(6H)-one

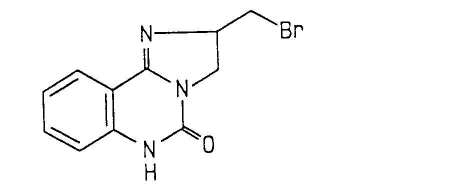

Method A: To a solution of 3,4-dihydro-4-imino-3-allyl-2(1H)-quinazolinone (10 g, 50 mmol) in tetrahydrofuran (70 mL) was added N-bromosuccinimide (9.0 g, 50 mmol). The mixture was then stirred at room temperature for 25 minutes. The solid was collected by filtration and recrystallized from THF to afford 2-bromomethyl-2,3-dihydro-imidazo[1,2-c]quinazolin-5(6H)-one (11.18 g, 95%), mp 213-214°C. ¹H NMR (300 MHz, DMSO-d₆): δ 3.67(m, 2H, CH₂), 3.74 (q, 1H, CH), 3.94 (t, 1H, CH), 4.57 (m, 1H, =CH), 7.07 (q, 2H, Ar-H), 7.51 (t, 1H, Ar-H), 7.78 (d, 1H, Ar-H), 10.65 (s, 1H, NH). ¹³C NMR (75 MHz, DMSO-d₆): d 47.80, 64.16, 110.27, 115.13, 122.23, 125.80, 133.59, 139.97, 147.47, 153.98, 206.07. Anal. Calcd for C₁₁H₁₀N₃OBr: C, 47.16; H, 3.59; N, 15.00. Found: C, 47.09; H, 3.41; N, 14.90.

Method B: To a solution of 3,4-dihydro-4-imino-3-allyl-2(1H)-quinazolinone (0.5 g, 2.5 mmol) in dimethylsulfoxide (2.5 mL) and water (1 mL) was added N-bromosuccinimide (0.87 g, 5.0 mmol). The mixture was allowed to stir at room temperature and then was quenched with water (100 mL) to get solid. The solid was collected by filtration and washed with water to give 2-bromomethyl-2,3-dihydroimidazo[1,2-c]quinazolin-5(6H)-one (450 mg, 64%).

Method C: To a suspension of 3,4-dihydro-4-imino-3-allyl-2(1H)-quinazolinone (0.5 g, 2.5 mmol) in water (30 mL) was added N-bromosuccinimide (443 mg, 2.5 mmol). The mixture was allowed to stir at room temperature for 30 minutes. The solid was collected by filtration and washed with water (10 mL) to afford 2-bromomethyl-2,3-dihydroimidazo[1,2-c]quinazolin-5(6H)-one (680 mg, 97%).

### Example 4

### 2-(4-phenyl-1-piperazinyl)methyl-2,3-dihydroimidazo[1,2-c]quinazolin-5(6H)-one

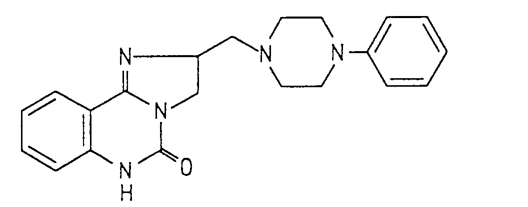

A mixture of 2-bromomethyl-2,3-dihydroimidazo[1,2-c]-quinazolin-5(6H)-one (0.5 g, 1.78 mmol), N-phenylpiperazine (0.28 mL, 3.56 mmol) in acetonitrile (25 mL) was refluxed for 13 hours. The hot mixture was filtered and washed with acetonitrile (10 mL) to give 2-(4-phenyl-1-piperazinyl)methyl-2,3-dihydroimidazo[1,2-c]-quinazolin-5(6H)-one (490 mg, 76%). An analytical sample was prepared from a mixture of acetonitrile and methanol (1:1), mp 257-258°C. ms:m/z 361 (M⁺). ¹H NMR (300 MHz, DMSO-d₆): δ 2.43-2.73 (m, 7H, CH₂), 3.11 (t, 3H, CH₂), 3.65 (q, 1H, CH), 3.91 (t, 1H, CH), 4.41 (m, 1H, CH), 6.75 (t, 1H, Ar-H), 6.91 (d, 2H, Ar-H), 7.06 (q, 2H, Ar-H), 7.17 (q, 2H, Ar-H), 7.48 (p, 1H, Ar-H), 7.78 (d, 1H, Ar-H), 10.53 (s, 1H, NH). Anal. Calcd for C₂₁H₂₃N₅O: C, 69.78; H, 6.41; N, 19.38. Found: C, 69.79; H, 6.45; N, 19.37.

### Example 5

### 3-(2,3-dihydroimidazo[1,2-c]quinazolin-5(6H)-one-2-yl)methyl-azaspiro[5,5]undecane

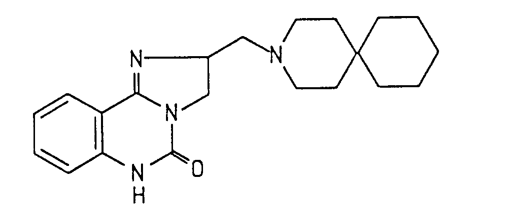

was obtained in 73% yield using a procedure similar to that which described in Example 4, mp 203-205°C, ms: m/z 166 (M⁺), ¹H NMR (300 MHz, DMSO-d₆): δ 1.27(s, 4H, CH₂), 1.34(s, 6H, CH₂), 2.28 (d, 2H, CH₂), 2.48 (d, 2H, CH₂), 3.39 (m, 7H, CH₂), 3.85 (t, 1H, CH), 4.34 (m, 1H, CH), 7.03 (m, 2H, Ar-H), 7.47 (t, 1H, Ar-H), 7.74 (d, 1H, Ar-H), 10.54 (s, 1H, NH). Anal. Calcd for C₂₁H₂₈N₄O: C, 71.56; H, 8.01; N, 15.90. Found; C, 71.50; H, 8.02; N, 15.89.

### Example 6

### 2-[4-piperonyl-1-piperazinyl]methyl-2,3-dihydroimidazo[1,2-c]quinazolin-5(6H)-one

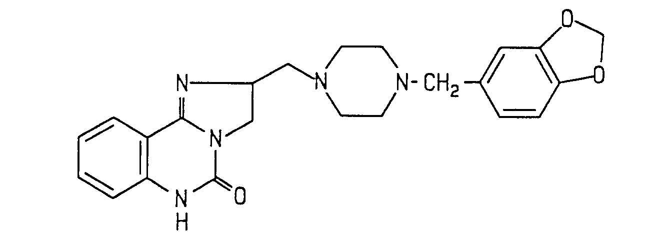

A mixture of 2-bromomethyl-2,3-dihydroimidazo[1,2-c]quinazolin-5(6H)-one (0.5 g, 1.78 mmol), 1-piperonylpiperazine (0.78 g, 3.56 mmol) in acetonitrile (25 mL) was refluxed for 17 hours. The solvent was then removed in vacuo to the oily residue. The residue was purified by column chromatography [silica gel: 25 g, column diameter: 2.5 cm, solvent system: chloroform/ethylacetate (9/1)] to furnish 2-[4-piperonyl-1-piperazinyl]methyl-2,3-dihydroimidazo[1,2-c]quinazolin-5(6H)-one hydrobromide (620 mg, 67%) and 2-methyl-2,3-dihydroimidazo[1,2-c]quinazolin-5(6H)-one (90 mg, 22%). 2-[4-piperonyl-1-piperazinyl]methyl-2,3-dihydroimidazo[1,2-c]quinazolin-5(6H)-one hydrobromide: mp 236-238°C. Anal. Calcd for C₂₃H₂₃N₅O₃/H₂O/HBr: C, 53.29; H, 5.44; N, 13.50. Found: C, 53.45; H, 5.46; N, 13.54.

### 2-methyl-2,3-dihydroimidazo[1,2-c]quinazolin-5(6H)-one

¹H NMR (300 MHz, DMSO-d₆): δ 2.32 (s, 3H, CH₃), 7.27 (t, 1H, Ar-H), 7.35 (d, 1H, Ar-H), 7.57 (s, 1H, Ar-H), 8.10 (d, 1H, Ar-H).Anal. Calcd for C₁₁H₉N₃O: C, 66.32; H, 4.55; N, 21.09. Found: C, 66.37; H, 4.56; N, 21.09.

### Example 7

### 2-[4-(2-methoxy)phenyl-1-piperazinyl]methyl-2,3-dihydroimidazo[1,2-c]quinazolin-5(6H)-one

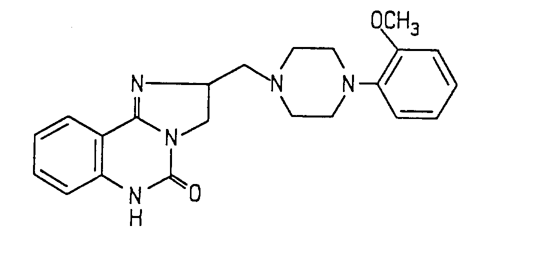

was obtained in 66% yield using a procedure similar to that which described in Example 4, mp 226-227°C. ms:m/z 391 (M⁺). ¹H NMR (300 MHz, DMSO-d₆): δ 2.44-2.69 (m, 6H, CH₂), 2.95 (br s, 4H, CH₂), 3.67 (q, 1H, CH), 3.76 (s, 3H, O-CH₃), 3.87 (t, 1H, CH), 4.40 (m, 1H, CH), 6.88 (d, 4H, Ar-H), 7.06 (m, 2H, Ar-H), 7.48 (t, 1H, Ar-H), 7.78 (d, 1H, Ar-H), 10.50 (br s, 1H, NH). Anal. Calcd for C₂₂H₂₅N₅O₂: C, 67.50; H, 6.44; N, 17.89. Found: C, 67.54; H, 6.44; N, 17.90.

### Example 8

### 2-[4-Benzyl-1-piperazinyl]methyl-2,3-dihydroimidazo[1,2-c]quinazolin-5(6H)-one

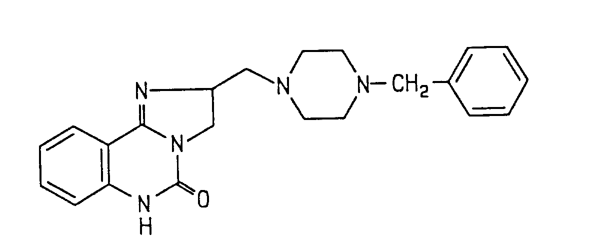

was obtained in 64% yield using a procedure similar to that which described in Example 4, mp 222-224°C. ¹H NMR (300 MHz, DMSO-d₆): δ 2.50-3.00 (br s, 10H, CH₂), 3.74-3.80 (br s, 3H, CH₂), 4.04 (t, 1H, CH), 4.53 (m, 1H, CH), 6.99-7.10 (m, 2H, Ar-H), 7.24-7.41 (m, 6H, Ar-H), 7.90 (d, 1H, Ar-H), 9.15 (br s, 1H, NH). Anal. Calcd for C₂₂H₂₅N₅O: C, 70.38; H, 6.71; N, 18.65. Found: C, 70.39; H, 6.67; N, 18.68.

### Example 9

### 2-[4-Benzyl-1-piperidinyl]methyl-2,3-dihydroimidazo[1,2-c]quinazolin-5(6H)-one

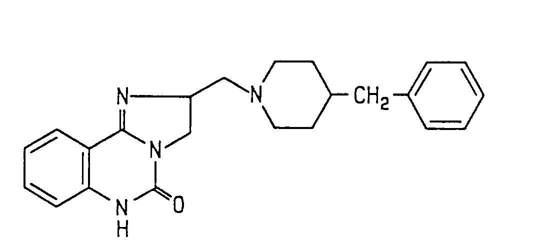

was obtained in 62% yield using a procedure similar to that which described in Example 4, mp 225-227°C. ¹H NMR (300 MHz, DMSO-d₆): δ 1.56 -1.66 (br s, 5H, CH₂), 2.21-2.33 (br s, 2H, CH₂), 2.54 (br s, 2H, CH), 2.75 (br s, 1H, CH), 2.90 (br s, 1H, CH), 3.09 (br s, 1H, CH), 3.29 (br s, 1H, CH), 3.80 (q, 1H, CH), 4.06 (t, 1H, CH), 4.60 (m, 1H, CH), 6.99-7.29 (m, 7H, Ar-H), 7.39 (t, 1H, Ar-H), 7.89 (d, 1H, Ar-H), 9.49 (br s, 1H, NH). Anal. Calcd for C₂₃H₂₆N₄O: C, 73.77; H, 7.00; N, 14.96. Found: C, 73.64; H, 7.02; N, 15.02.

### Example 10

### 2-[4-(2-Pyridinyl)-1-piperazinyl]methyl-2,3-dihydroimidazo[1,2-c]quinazolin-5(6H)-one

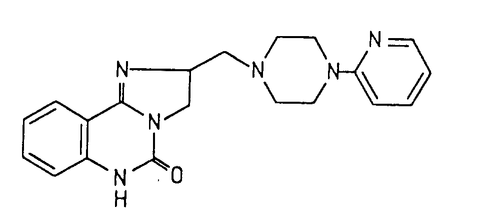

was obtained in 81% yield using a procedure similar to that which described in Example 4, mp 242-243°C. ¹H NMR (300 MHz, DMSO-d₆): δ 2.65-2.83 (br s, 6H, CH₂), 3.59 (br s, 4H, CH₂), 3.91 (q, 1H, CH), 4.09 (t, 1H, CH), 4.57 (m, 1H, CH), 6.62 (t, 2H, Ar-H), 6.98 (d, 1H, Ar-H), 7.11 (t, 1H, Ar-H), 7.45 (q, 2H, Ar-H), 7.95 (d, 1H, Ar-H), 8.17 (d, 1H, Ar-H), 9.04 (br s, 1H, NH). Anal. Calcd for C₂₀H₂₂N₆O: C, 66.28; H, 6.12; N, 23.19. Found: C, 66.23; H, 6.10; N, 23.1.

### Example 11

### 2-[4-(4-fluorobenzyl)-1-piperazinyl]methyl-2,3-dihydroimidazo[1,2-c]quinazolin-5(6H)-one

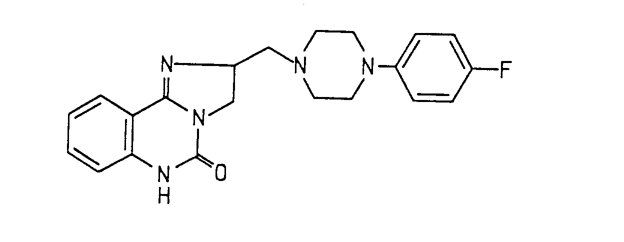

was obtained in 61% yield using a procedure similar to that which described in Example 4, mp 248-249°C. Anal. Calcd for C₂₁H₂₂N₅OF: C, 66.48; H, 5.84; N, 18.46. Found: C, 66.54; H, 5.85; N, 18.49.

### Example 12

### 2-[1-benzylpiperidin-4-yl]aminomethyl-2,3-dihydroimidazo[1,2-c]quinazolin-5(6H)-one

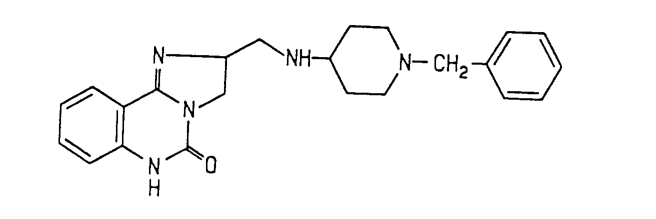

was obtained in 76% yield using a procedure similar to that which described in Example 4, mp 206-208°C. Anal. Calcd for C₂₃H₂₇N₅₀: C, 70.93; H, 6.99; N, 17.98. Found: C, 70.71; H, 6.87; N, 17.97.

### Example 13

### 8-(2,3-dihydroimidazo[1,2-c]quinazolin-5(6H)-one-2-yl)methyl-1-phenyl-1,3,8-triazaspiro[4,5]decan-4-one

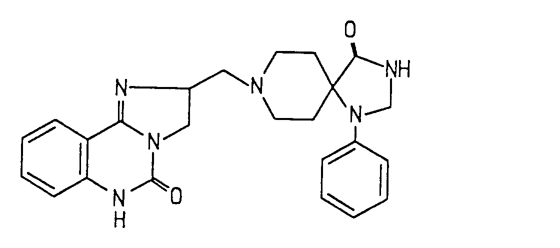

was obtained in 74% yield using a procedure similar to that which described in Example 4, mp 250-251°C (acetonitrile); ¹H NMR (300 MHz, DMSO-d₆): δ 1.52 (q, 2H, CH₂), 2.44-2.64 (m, 4H, 2 CH₂), 2.78-2.89 (m, 4H, 2CH₂), 3.71-3.77 (m, 1H, CH), 3.96 (m, 1H, CH), 4.37 (m, 1H, CH), 4.55 (s, 2H, CH₂), 6.71-6.82 (m, 3H, Ar-H), 7.06 (t, 2H, Ar-H), 7.22 (t, 2H, Ar-H), 7.46 (t, 1H, Ar-H), 7.77 (d, 1H, Ar-H), 8.59 (s, 1H, NH), 10.54 (s, 1H, NH). Anal. Calcd for C₂₄H₂₆N₆O₂. 1/4H₂O (430.508): C, 66.27; H, 6.14; N, 19.32. Found: C, 66.32; H, 6.14; N, 19.21

### Example 14

### 2-[1-(4-chlorobenzhydryl)piperazinyl]methyl-2,3-dihydroimidazo[1,2-c]quinazolin-5(6H)-one

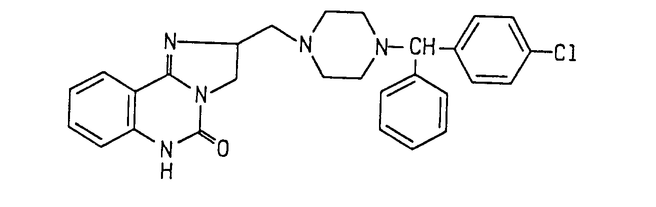

was obtained in 40% yield using a procedure similar to that which described in Example 4, mp 245-246°C (acetonitrile); ¹H NMR (300 MHz, DMSO-d₆): δ 2.29-2.57 (m, 10H, CH₂), 3.29 (m, 1H, CH), 3.59 (m, 1H, CH), 3.84 (t, 1H, CH), 4.31 (s, 2H, CH₂), 7.01-7.75 (m, 13 H, Ar-H), 10.50 (br s, 1H, NH). Anal. Calcd for C₂₈H₂₉N₅OCl (486.015): C, 69.20; H, 5.81; N, 14.40. Found: C, 68.85; H, 5.84; N, 14.26.

### Example 15

### 2-[1-(4-p-fluorobenzoyl)piperidinyl]methyl-2,3-dihydroimidazo[1,2-c]quinazolin-5(6H)-one

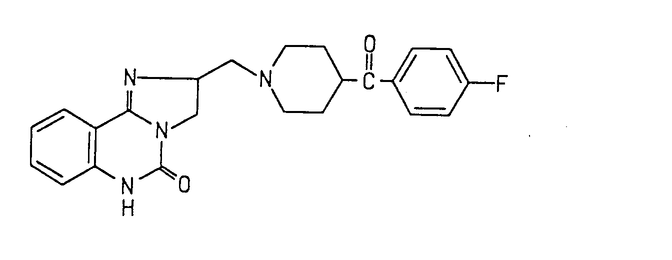

was obtained in 81% yield using a procedure similar to that which described in Example 4, mp 220-221°C (acetone); ¹H NMR (300 MHz, DMSO-d₆): δ 1.51-1.73 (m, 4H, 2 CH₂), 2.10-2.42 (m, 5H, 2 CH₂ + CH), 2.87 (d, 1H, CH), 3.06 (d, 1H, CH), 3.36 (m, 1H, CH), 3.62 (m, 1H, CH), 3.88 (m, 1H, CH), 4.37 (m, 1H, CH), 7.06 (m, 2H, Ar-H), 7.33 (m, 2H, Ar-H), 7.76 (d, 1H, Ar-H), 8.04 (m, 2H, Ar-H), 10.50 (s, 1H, NH).

### Example 16

### 2-[1-(4-methoxyphenyl)piperazinyl]methyl-2,3-dihydroimidazo[1,2-c]quinazolin-5(6H)-one

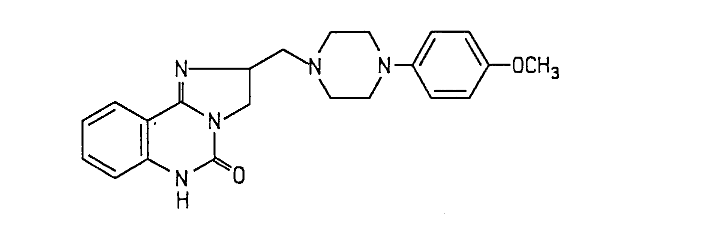

was obtained in 54% yield using a procedure similar to that which described in Example 4, mp 229-231°C (CH₃CN); ¹H NMR (300 MHz, DMSO-d₆): δ 2.42-2.71 (m, 6H, 3CH₂), 3.09 (m, 4H, 2CH₂), 3.65 (m, 1H, CH), 3.69 (s, 3H, CH₃), 3.90 (m, 1H, CH), 4.41 (m, 1H, CH), 6.33-6.51 (m, 3H, Ar-H), 7.03-7.11 (m, 3H, Ar-H), 7.48 (t, 1H, Ar-H), 7.78 (d, 1H, Ar-H), 10.50 (s, 1H, NH); ms; m/z 391 (M⁺), 229, 205. Anal. Calcd for C₂₂H₂₅N₅O₂ (391.475): C, 67.50; H, 6.44; N, 17.89. Found: C, 67.67; H, 6.36; N, 17.82.

### Example 17

### 2-[1-(3-methoxyphenyl)piperazinyl]methyl-2,3-dihydroimidazo[1,2-c]quinazolin-5(6H)-one

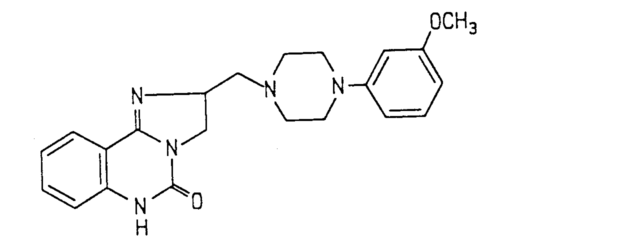

was obtained in 88% yield using a procedure similar to that which described in Example 4, mp >300°C. Anal. Calcd for C₂₂H₂₅N₅O₂ (391.475): C, 67.50; H, 6.44; N, 17.89. Found: C, 67.47; H, 6.31; N, 17.72.

### Example 18

### 3,4-dihydro-4-imino-3-allyl-quinazolin-2(1H)-thione

A mixture of anthranilonitrile (10.0 g, 85 mmol) and allyl isothiocyanate (15 mL, 151 mmol) was slightly heated to dissolve. The resulting solution was allowed to stir at room temperature for five days. The solid was collected by filtration and washed with acetonitrile (10 mL), and was recrystallized from ethanol to give 8.05 g (44%) of 3,4-dihydro-4-imino-3-allyl-quinazolinone-2(1H)-thione, mp 168-169°C. ¹H NMR (300 MHz, DMSO-d₆): δ 5.07-5.25 (m, 4H, =CH₂ + CH₂), 5.75-6.06 (m, 1H, =CH), 7.13-7.63 (m, 3H, Ar-H), 8.05-8.13 (d, 1H, Ar-H), 9.40 (s, 1H, NH), 12.13 (s, 1H, NH). ¹³C-NMR (75 MHz, DMSO-d₆): δ 48.43, 115.56, 116.63, 123.92, 125.96, 132.20, 133.04, 135.59, 174.38.ms: m/z, 217 (M⁺), 201, 183, 160. Anal. Calcd for C₁₁H₁₁N₃S (217.29); C, 60.80; H, 5.10; N, 19.34. Found: C, 60.95; H, 5.13; N, 19.34.

### Example 19

### 2-bromomethyl-2,3-dihydroimidazo[1,2-c]quinazolin-5(6H)-thione

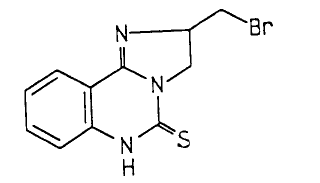

To a solution of 3,4-dihydro-4-imino-3-allyl-quinazolin-2(1H)-thione (5.5 g, 25.31 mmol) in acetonitrile (60 mL) was added N-bromosuccinimide (6.0 g, 33.52 mmol). The mixture was then stirred at room temperature for 50 minutes. The solid was collected by filtration and recrystallized from a mixture of acetonitrile and methanol to afford 2-bromomethyl-2,3-dihydro-imidazo[1,2-c]quinazolin-5(6H)-thione (3.01 g, 40%), mp >300°C. ¹H NMR (300 MHz, DMSO-d₆): δ 3.90(m, 2H, CH₂), 4.54-4.73(m, 3H, CH & CH₂), 7.66.7.71 (t, 2H, Ar-H), 7.97-9.02 (m, 1H, Ar-H), 8.54 (d, 1H, Ar-H), 9.80 (s, 1H, NH), 10.50 (s, 1H, NH). ¹³C-NMR (100 MHz, DMSO-d₆): δ 47.80, 64.16, 110.27, 115.13, 122.23, 125.80, 133.59, 139.97, 147.47, 153.98, 206.07. ms:m/z, 296 (M⁺), 295 (M⁺-1), 216(M⁺-80), 202(M⁺-94). Anal. Calcd for C₁₁H₁₀N₃SBr · HBr (377.10): C, 35.04; H, 2.94; N, 11.14. Found: C, 35.14; H, 3.01; N, 10.94.

### Example 20

### 2-(4-phenyl-1-piperazinyl)methyl-2,3-dihydroimidazo[1,2-c]quinazolin-5(6H)-thione

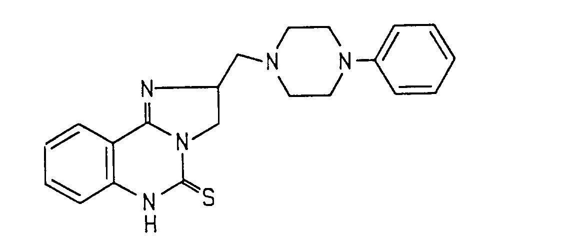

A mixture of 2-bromomethyl-2,3-dihydroimidazo[1,2-c]quinazolin-5(6H)-thione (3.0 g, 10.13 mmol), N-phenylpiperazine (2.5 mL, 15.41 mmol) and sodium bicarbonate (1.7 g, 20.24 mmol) in acetonitrile (70 mL) was refluxed for 24 hours. The hot mixture was filtered and washed with acetonitrile (10 mL) to give 2-(4-phenyl-1-piperazinyl)methyl-2,3-dihydroimidazo[1,2-c]quinazolin-5(6H)-thione (490 mg, 76%). An analytical sample was prepared from a mixture of acetonitrile and methanol (1:1), mp 257-258°C. ms:m/z 361 (M⁺). ¹H NMR (300 MHz, DMSO-d₆): δ 2.43-2.73 (m, 7H, CH₂), 3.11 (t, 3H, CH₂), 3.65 (q, 1H, CH), 3.91 (t, 1H, CH), 4.41 (m, 1H, CH), 6.75 (t, 1H, Ar-H), 6.91 (d, 2H, Ar-H), 7.06 (q, 2H, Ar-H), 7.17 (q, 2H, Ar-H), 7.48 (p, 1H, Ar-H), 7.78 (d, 1H, Ar-H), 10.53 (s, 1H, NH). ¹³C NMR (75 MHz, DMSO-d₆): δ 29.43, 48.14, 51.91, 52.67, 61.18, 115.33, 118.75, 124.75, 124.92, 125.60, 128.81, 132.41, 145.39, 150.86, 159.50, 179.22.

### Example 21

### 2-nitro-4,5-dimethoxybenzonitrile

To a nitric acid (40 mL) in ice bath was added 3,4-dimethoxybenzonitrile (5.0 g, 30.6 mmol) and was allowed to stir for 2 hours. Then the solution was gradually returned to room temperature. To this solution was added ice (200 mL) to get light yellow precipitate which was then collected by filtration to give 6.25 g (98%) of 2-nitro-4,5-dimethoxybenzonitrile. ¹H NMR (100 MHz, DMSO-d₆): δ 3.69 (s, 3H, CH₃), 3.90 (s, 3H, CH₃), 7.53 (s, 1H, Ar-H), 7.75 (s, 1H, Ar-H). ms: m/z 208 (M⁺), 192 (M⁺-16), 178 (M⁺-30), 162 (M⁺-46).

### Example 22

### 2-amino-4,5-dimethoxybenzonitrile

A suspension of 2-nitro-4,5-dimethoxybenzonitrile (6.0 g, 28.8 mmol) in water (200 mL) was heated to 90 °C. To this hot mixture was added sodium dithionite in a small portion until it was dissolved completely. The resulting solution was filtered and then cooled to room temperature to get crystal. The solid was collected by filtration to afford 4.99 g (97%) of 2-amino-4,5-dimethoxybenzonitrile. An analytical sample was recrystallized from acetonitrile. ¹H NMR (100 MHz, DMSO-d₆): δ 3.64 (s, 3H, CH₃), 3.73 (s, 3H, CH₃), 4.32 (br s, 2H, NH₂), 6.41 (s, 1H, Ar-H), 6.86 (s, 1H, Ar-H). ¹³C NMR (25 MHz, DMSO-d₆): d 55.55, 56.87, 83.67, 92.08, 113.96, 140.33, 148.12, 154.33. MS: m/z 178 (M⁺), 163 (M⁺-15), 147 (M⁺-31), 135 (M⁺-43), 120 (M⁺-58).

### Example 23

### 3,4-dihydro-3-allyl-4-imino-6,7-dimethoxyquinazolin-2(H)-one

was prepared in 72% yield using a similar procedure which described in Example 2. An analytical sample was recrystallized from ethanol. ¹H NMR (100 MHz, DMSO-d₆): δ 3.78 (s, 6H, 2(CH₃)), 4.62 (d, 2H, J=4.8 Hz, CH₂), 5.05 (d, 2H, J=12.8 Hz, CH₂), 5.94-6.03 (m, 1H, CH), 6.62 (s, 1H, Ar-H), 7.54 (s, 1H, Ar-H). ms: m/z 260 (M⁺-1), 246 (M⁺-15), 230 (M⁺-31), 216 (M⁺-45), 206 (M⁺-55).

### Example 24

### 2-bromomethyl-8,9-dimethoxy-2,3-dihydroimidazo[1,2-c]quinazolin-5(6H)-one

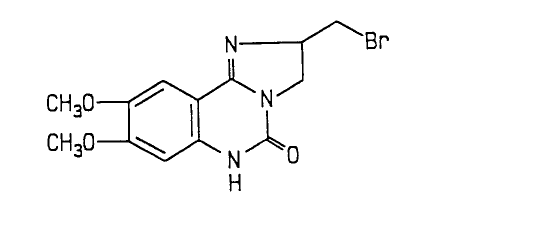

was prepared in 92% yield using a similar procedure as described in Example 3. ¹H NMR (100 MHz, DMSO-d₆): δ 3.78 (s, 3H, CH₃), 3.83 (s, 3H, CH₃), 3.78-4.21 (m, 4H, 2 (CH₂)), 6.68 (s, 1H, Ar-H), 7.32 (s, 1H, Ar-H). MS: m/z 341 (M⁺), 259 (M⁺-82), 246 (M⁺-95).

### Example 25

### 2-(4-phenyl-1-piperazinyl)methyl-8,9-dimethoxy-2,3-dihydro-imidazo[1,2-c]quinazolin-5(6H)-one

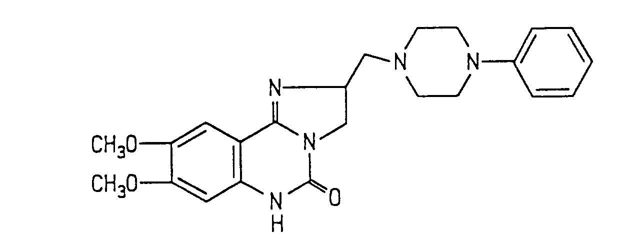

was obtained in 90% yield using a procedure similar to that which described in Example 4. An analytical sample was recrystallized from ethanol, mp 283-284 °C, ms: m/z 421 (M⁺), 303 (M⁺-118), 289 (M⁺-132), 275 (M⁺-146). ¹H NMR (100 MHz, DMSO-d₆ + 1N D₂SO₄): δ 3.37 (brs, 8H, 4(CH₂)), 3.81 (s, 3H, CH₃), 3.88 (s, 3H, CH₃), 3.62-4.20 (m, 3H, CH₂ + CH), 4.46 (t, 1H, CH), 4.95 (br s, 1H, CH), 6.79 (s, 1H, Ar-H), 6.82-7.23 (m, 5H, Ar-H), 7.56 (s, 1H, Ar-H). ¹³C-NMR (25 MHz, DMSO-d₆ + 1N D₂SO₄): δ 46.35, 49.70, 52.15, 52.50, 56.78, 57.19, 58.82, 96.33, 98.55, 107.05, 116.66, 121.11, 130.22, 141.02, 146.02, 146.66, 148.53, 158.37, 158.55. Anal. Calcd for C₂₃H₂₇N₅O₃ (421.50): C, 65.54; H, 16.62; N, 6.46. Found: C, 65.33; H, 16.54; N, 6.51.

### Example 26

### 2-[4-(2-chlorophenyl)-1-piperazinyl]methyl-2,3-dihydroimidazo[1,2-c]quinazolin-5(6H)-one

To a mixture of 2-chloromethyl-2,3-dihydroimidazo[1,2-c] quinazolin-5(6H)-one (1.0 g, 3.6 mmol) in acetonitrile (20 mL) was added 1-(2-chlorophenyl)piperazine hydrochloride (1.3 g, 5.4 mmol) and and sodium hydrocarbonate (0.5 g, 5.4 mmol). The mixture was allowed to reflux at 90°C for 48 hours. The white solid was collected by filtration after the mixture was cooled to room temperature and was recrystallized from ethanol to afford 0.95 g (67% yield) of title compound, m.p. 235-236°C. ¹H-NMR (400 MHz, DMSO-d₆): δ 2.41-2.84 (m, 6H, 3CH₂), 2.92-3.02 (m, 2H, CH₂), 3.07-3.31 (m, 2H, CH₂), 3.67-4.03 (m, 2H, CH₂), 4.22-4.28 (m, 1H, CH), 7.34-8.13 (m, 8H, Ar-H), 10.89 (s, 1H, NH); ¹³C-NMR (100 MHz, DMSO-d₆): δ 48.01, 50.93, 53.40, 62.91, 62.99, 111.28, 115.14, 120.85, 122.18, 123.81, 125.79, 127.62, 128.05, 130.30, 133.16, 139.99, 148.26, 149.05, 152.69; ms: m/z 394 (M⁺). Anal. Calcd for C₂₁H₂₂N₅OCl (395.89): C, 63.71; H, 5.60; N, 17.69. Found: C, 63.35; H, 5.51; N, 17.64.

### Example 27

### 2-(4-phenyl-1-piperazinyl)methyl-6-methyl-2,3-dihydroimidazo[1,2-c]quinazolin-5-one

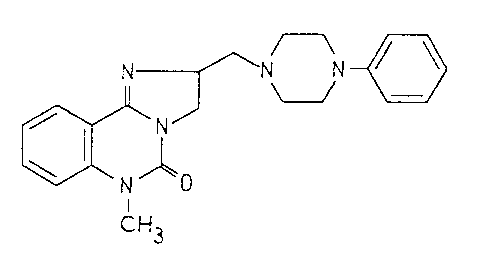

To a solution of 2-(4-phenyl-1-piperazinyl)methyl-2,3-dihydroimidazo[1,2-c]quinazolin-5(6H)-one (1.0 g, 2.8 mmol) in DMF (30 mL) was added 50% sodium hydride (0.27 g, 5.6 mmol). The solution was stirred at room temperature for 1 hour and then to the solution was added methyl iodide (0.21 mL, 3.36 mmol). The resulting solution was stirred at room temperature and after 24 hours, the mixture was poured into ice water (100 mL) and the mixture was then set in refrigerator overnight to get white precipitates which was collected by filtration to give title compound (0.4 g, 38%). An analytical sample was recrystallized from acetonitrile, m.p. 185-186°C. ¹H-NMR (400 MHz, CDCl₃): δ 2.41- 2.75 (m, 6H, 3CH₂), 3.11 (m, 4H, 2CH₂), 3.39 (s, 3H, CH₃), 3.84-3.88 (m, 1H, CH), 3.99-4.04 (m, 1H, CH), 4.37-4.45 (m, 1H, CH), 6.76 (t, J= 7.8 Hz, 1H, Ar-H), 7.18 (m, 2H, Ar-H), 7.46 (t, J=7.3 Hz, 1H, Ar-H), 7.97 (d, J=7.9 Hz, 1H, Ar-H); ¹³C-NMR (100.40 MHz, CDCl₃): δ 29.69, 49.11, 49.62, 53.79, 63.38, 112.86, 113.87, 115.97, 119.56, 122.67, 126.88, 129.01, 133.42, 140.88, 148.96, 151.27, 152.88; ms: m/z 375 (M⁺). Anal. Calcd for C₂₂H₂₅N₅O(375.48): C, 70.38; H, 6.71; N, 18.65. Found: C, 70.70; H, 6.74; N, 18.58.

### Example 28

### 2-[4-(2-chlorophenyl)-1-piperazinyl]methyl-6-methyl-2,3-dihydroimidazo[1,2-c]quinazolin-5-one

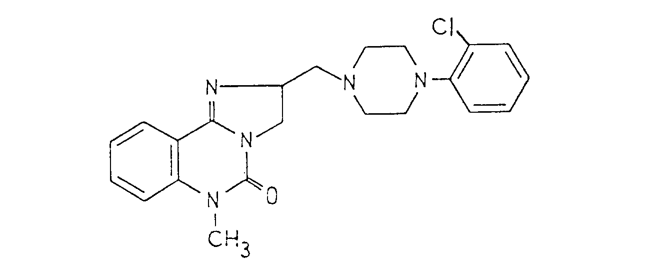

To a solution of 2-[4-(2-chlorophenyl)-1-piperazinyl]methyl-2,3-dihydroimidazo[1,2-c]quinazolin-5(6H)-one (1.0 g, 2.5 mmol) in DMF (20 mL) was added 50% sodium hydride (0.24 g, 5.0 mmol). The solution was stirred at rooom temperature for 1 hour and then to the solution was added methyl iodide (0.2 mL, 4.56 mmol). The resulting solution was stirred at room temperature and after 24 hours, the mixture was poured into ice water (200 mL) to get white precipitates which was collected by filtration to give title compound (0.92 g, 90%). An analytical sample was recrystallized from acetonitrile, m.p. 157-158 °C. ¹H-NMR (400 MHz, DMSO-d₆): δ 2.43-2.73 (m, 6H, 3CH₂), 2.97 (m, 4H, 2CH₂), 3.34 (s, 3H, CH₃), 3.70 (m, 1H, CH), 3.92 (m, 1H, CH), 4.40 (m, 1H, CH), 7.00-7.89 (m, 8H, Ar-H); ¹³C-NMR (100.40 MHz, DMSO-d₆): δ 29.39, 47.97, 49.17, 50.89, 53.34, 62.70, 62.87, 111.24, 112.36, 114.61, 115.08 120.79, 122.13, 122.38, 123.75 125.74, 125.93, 127.58, 128.01, 130.26, 133.12, 133.43, 139.96, 140.67, 148.22, 149.01, 151.73, 152.65; ms: m/z 410 (M⁺ +1), 409 (M⁺), 395 (M⁺-14), 282 (M⁺-137), 257 (M⁺-152), 209 (M⁺-200, 100%). Anal. Calcd for C₂₂H₂₄N₅OCl (409.92): C, 64.46; H, 5.90; N, 17.09. Found: C, 64.08; H, 5.83; N, 17.30.

## Claims

1. An imidazo[1,2-c]quinazoline compound having the formula 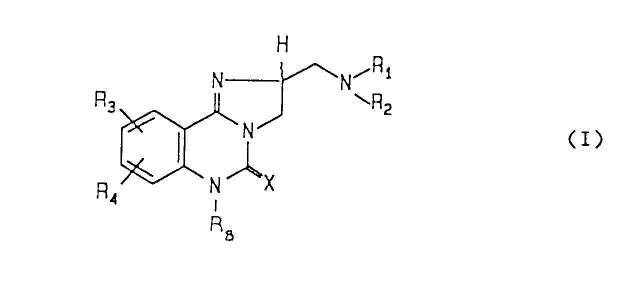 which comprises racemic mixture of optically active compounds or optically pure R and S stereoisomers,
wherein:
X is O or S;
R₁ is hydrogen, lower alkyl, phenyl or phenyl(lower alkyl);
R₂ is hydrogen, lower alkyl, phenyl or phenyl(lower alkyl); or R₁ and R₂ together represent alkylene of 4 to 10 carbon stoms or 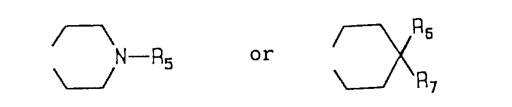 wherein R₅ represents (a) 2-(2-hydroxyethoxy)ethyl; (b) pyrimidinyl; (c) a 2-, 3- or 4-pyridinyl optionally substituted by one or two substituents each independendly selected from the group consisting of C₁-C₄ alkyl; and (d) benzoyl, benzyl, phenyl and diphenylmethyl optionally substituted by one or two substituents selected from the group consisting of halogen atom, methoxy, trifluoromethyl, C₁-C₄ alkyl, nitro, acetyl, and cyano; and
(i) R₆ and R₇ together represent alkylene of 4 to 6 carbon atoms or
(ii) 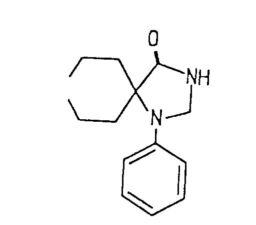
or (iii) when R₆ represents hydrogen atom or hydroxy, R₇ represents (a) pyrimidinyl; (b) a 2-, 3- or 4-pyridinyl optionally substituted by one or two substituents each independendly selected from the group consisting of C₁-C₄ alkyl; and (c) benzoyl, benzyl, phenyl and diphenylmethyl optionally substituted by one or two substituents selected from the group consisting of halogen atom, methoxy, trifluoromethyl, C₁-C₄ alkyl, nitro, acetyl, and cyano;
R₃ is halogen, hydrogen, alkyl, trifluoromethyl, or lower alkoxy;
R₄ is halogen, hydrogen, alkyl, trifluoromethyl, or lower alkoxy; and
R₈ is hydrogen, C₁-C₄ alkyl or phenyl(C₁-C₄)alkyl; or pharmaceutically acceptable salts thereof.

2. A process for preparing the compound of formula (I) as defined in claim 1 comprising
(a) reacting a compound having the formula 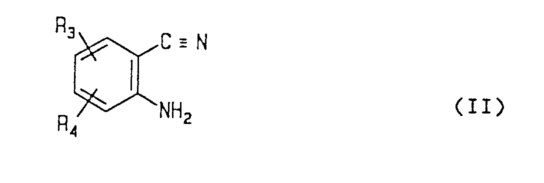 wherein R₃ and R₄ have the same meanings as defined in claim 1, with a compound of the formula 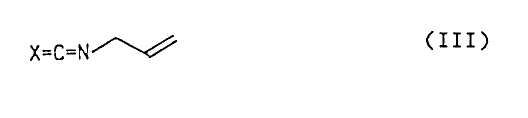 wherein X has the same meaning as defined in claim 1;
(b) reacting the resulting compound having the formula 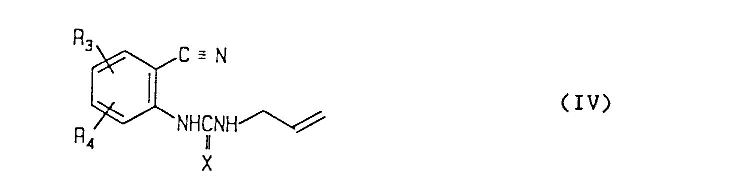 wherein R₃, R₄ and X have the same meanings as defined above, with a base;
(c) reacting the resulting compound having the formula 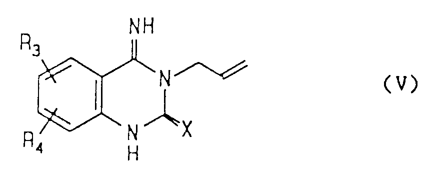 wherein R₃, R₄ and X have the same meanings as defined above, with N-halosuccimide or halogen;
(d) reacting the resulting compound having the formula 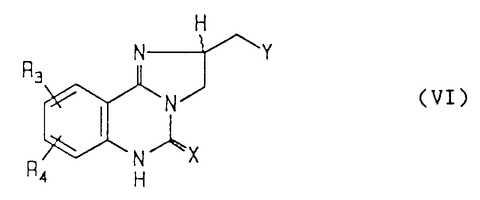 wherein R₃, R₄ and X have the same meanings as defined above; Y represents halogen,
with a compound of formula 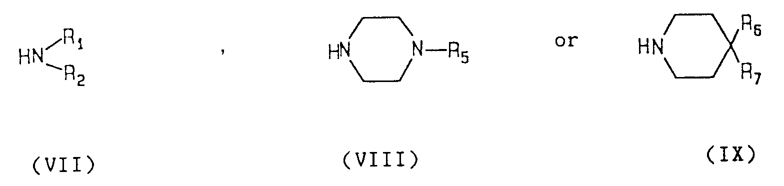 wherein R₁, R₂, R₅, R₆ and R₇ have the same meanings as defined in claim 1, as a suitable side chain, to obtain 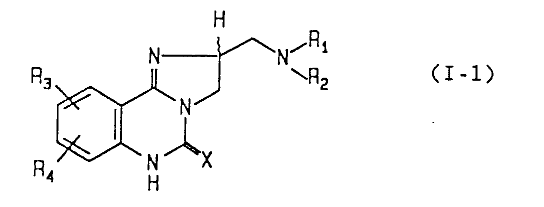 wherein R₁, R₂, R₃, and R₄ have the same meanings as defined above; and
(e) reacting the resulting compound (I-1) with an alkylating agent of formula R₈-Y to obtain 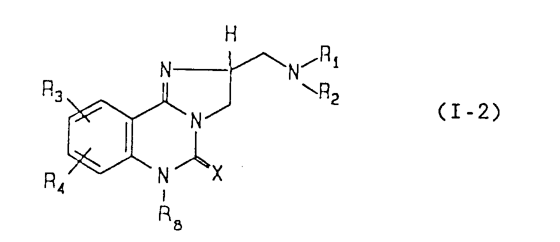 wherein R₈ is C₁-C₄ alkyl or phenyl(C₁-C₄)alkyl; Y is halogen; and R₁, R₂, R₃, and R₄ have the same meanings as defined above.

3. A pharmaceutical composition for the treatment of hypertension, which comprises a therapeutically effective amount of an imidazo[1,2-c]quinazoline compound of the formula (I) as defined in claim 1 or a pharmaceutically acceptable salt thereof, as an active ingredient, in combination with a pharmaceutically acceptable carrier or diluent for the active ingredient.

4. A method for the treatment of hypertension comprising administrating a therapeutically effective amount of an imidazo[1,2-c]quinazoline compound of the formula (I) as defined in claim 1 to a subject suffering from high blood pressure.

5. A pharmaceutical composition for the treatment of dysuria, which comprises a therapeutically effective amount of an imidazo[1,2-c]quinazoline compound of the formula (I) as defined in claim 1 or a pharmaceutically acceptable salt thereof, as an active ingredient, in combination with a pharmaceutically acceptable carrier or diluent for the active ingredient.

6. A method for the treatment of dysuria comprising administrating a therapeutically effective amount of an imidazo[1,2-c]quinazoline compound of the formula (I) as defined in claim 1 to a subject suffering from prostatauxe.

7. An imidazo[1,2-c]quinazoline compound or a pharmaceutically acceptable salt thereof, as claimed in claim 1, which is selected from a class of compounds consisting of:
(4-phenyl-1-piperazinyl)methyl-2,3-dihydroimidazo[1,2-c]quinazolin-5(6H)-one; 2-(4-phenyl-1-piperazinyl)methyl-2,3-dihydroimidazo[1,2-c]quinazolin-5(6H)-thione; 2-(4-phenyl-1-piperazinyl)methyl-8,9-dimethoxy-2,3-dihydro-imidazo[1,2-c]quinazolin-5(6H)-one;
2-[4-(2-chlorophenyl)-1-piperazinyl]methyl-2,3-dihydroimidazo[1,2-c]quinazolin-5(6H)-one; 2-(4-phenyl-1-piperazinyl)methyl-6-methyl-2,3-dihydroimidazo[1,2-c]quinazolin-5-one; and 2-[4-(2-chlorophenyl)-1-piperazinyl]methyl-6-methyl-2,3-dihydroimidazo[1,2-c]quinazolin-5-one.
